(19) 

Europäisches
Patentamt

European
Patent Office

Office européen
des brevets

(11)  **EP 3 937 843 B1**

(12)  **EUROPEAN PATENT SPECIFICATION**

(45) Date of publication and mention
of the grant of the patent:
**06.11.2024  Bulletin 2024/45**

(21) Application number: **20711119.6**

(22) Date of filing: **10.03.2020**

(51) International Patent Classification (IPC):
***A61C 13/15*** ^(2006.01)

(52) Cooperative Patent Classification (CPC):
**A61C 19/004; A61B 1/126; A61B 1/253;
A61B 5/0071; A61B 5/0088; A61B 5/0534;
A61C 5/40; A61C 5/55**

(86) International application number:
**PCT/EP2020/056396**

(87) International publication number:
**WO 2020/182837 (17.09.2020 Gazette 2020/38)**

(54) **DENTAL INSTRUMENT**

ZAHNMEDIZINISCHES INSTRUMENT

INSTRUMENT DENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **11.03.2019  EP 19162053**

(43) Date of publication of application:
**19.01.2022  Bulletin 2022/03**

(73) Proprietor: **I-DENT Innovations for Dentistry SA
1260 Nyon (CH)**

(72) Inventor: **BEERSTECHER, Jan
1532 Fétigny (CH)**

(74) Representative: **Wächtershäuser & Hartz
Patentanwaltspartnerschaft mbB
Weinstraße 8
80333 München (DE)**

(56) References cited:
**US-A1- 2005 282 102     US-A1- 2006 275 732
US-A1- 2012 230 017     US-B1- 9 585 549**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

**Field of the Invention**

The present invention relates to a modular cord-less pen-style dental instrument comprising an elongated body for gripping and holding the instrument, which may be combined with a wand portion having a specific functionality. Different wand portions providing different functionalities may be employed. Accordingly, the present invention also provides a kit-of-parts for providing a modular cord-less pen-style dental instrument of the present invention, which comprises at least two wand portions.

**Background of the Invention**

A dental practitioner may encounter a wide range of dental conditions requiring treatment. For example, a dental patient may require preventive, therapeutic diagnostic or cosmetic treatment. In order to be able to treat each of the different conditions, the dental practitioner is required to keep a wide range of dental instruments available and ready for use, including dental handpieces for rotary tools which may be connected to an external control unit and/or power supply. Dental handpieces are useful in that different rotary tools may be connected to the dental handpiece. However, the dental practitioner requires additional functionality which goes beyond the capabilities of a dental handpiece. For this purpose, the dental practitioner may use additional specialized instruments, each provided and limited to a specific functionality.

For example, for curing photocurable compositions, the dental practitioner uses a dental curing light comprising a light source adapted to emit radiation of a specific wavelength. However, different photocurable dental compositions may require light of a different wavelength for curing. Accordingly, the dental practitioner needs as many dental curing lights as potentially different photocurable dental compositions need to be cured in the future. Moreover, the illumination of dental tissue may be required for reasons other than photocuring a dental composition whereby the wavelength and intensity of the light must again be adapted to the specific functionality.

Finally, the dental practitioner may require further dental instruments which are unrelated to any irradiation functionality. For example, the dental practitioner may require a dental mirror which rotates in order to prevent blurring by water spray or saliva when the dental mirror is introduced into the buccal cavity. Moreover, certain highly viscous dental compositions are provided with thixotropic properties so that the application of ultrasound vibration facilitates the flow of the dental composition into a dental cavity. Accordingly, the dental practitioner requires an instrument which is adapted to provide ultrasonic vibrational capabilities for this specific type of dental composition.

The need to own a different dental instrument for each of the different functionalities which are required by the dental practitioner to treat a wide range of dental conditions may be quite costly. Moreover the maintenance of a large selection of different dental instruments requires additional resources. In view of the continuing diversification of the dental products and therapies, it is desirable to combine functionalities in a single instrument.

For example, US2006275732 discloses curing lights which can be constructed using more than one LED, where at least 2 of the LEDs have different spectral profiles in order to have the capability to activate initiators sensitive to light of different wavelengths.

However, the dental instruments known from the prior art, in particular cord-less pen-style dental instruments providing irradiation functionality of light with different wavelength as cited above cannot be used for providing unrelated and diverse functionalities such as providing self-cleaning indirect vision by rotating a dental mirror, vibrating a dental tip, providing pulp vitality testing based on current stimulation or heat/cold application, or providing heated Gutta percha plugging.

US 2005/282102 A1 discloses a kit for use by dental professionals comprising a handle part with a proximal end and a distal end and a plurality of user interchangeable electrically operated heads to be attached to the distal end of said handle part. The handle part provides power for any of the heads fitted thereto.

**Summary of the Invention**

According to a first aspect, it is a problem of the present invention to provide a cord-less pen-style dental instrument which may be used for providing functionality in the treatment of a wide range of dental conditions whereby the functionality may be selected from a wide range of alternative functionalities including irradiation of light of a specific wave length or wave length range, providing self-cleaning indirect vision by rotating a dental mirror, vibrating a dental tip, providing pulp vitality testing based on current stimulation or heat/cold application, or providing heated Gutta percha plugging.

According to a second aspect, it is a further problem of the present invention to provide a kit-of-parts for providing a modular cord-less pen-style dental instrument as defined by the present invention which may be used for providing functionality in the treatment of a wide range of dental conditions whereby the functionality may be selected from a wide range of alternative functionalities including irradiation of light of a specific wave length or wave length range, providing self-cleaning indirect vision by rotating a dental mirror, vibrating a dental tip, providing pulp vitality testing based on current stimulation or heat/cold application, or providing heated Gutta percha plugging.

According to the first aspect, the present invention provides a modular cord-less pen-style dental instrument comprising:

> (a) an elongated body for gripping and holding the instrument, which has a connector portion at the distal end thereof;
> (b) a wand portion having a proximal end adapted to be releasably connected to the connector portion at the distal end of the elongated body;
>
> wherein the connector portion provides thermal insulation via a heat flow barrier (30) between the wand portion (20) and the elongated body (10) to reduce the accumulation of heat in the elongated body (10);
> wherein the elongated body (10) comprises a rechargeable battery (111) and a main control unit (67) communicatively connected through the connector portion (12) with the wand portion (20), and
> the wand portion (20) comprises a module control unit (21) powered by the rechargeable battery (111) in the elongated body (10) and communicatively connected with the main control unit (67) of the elongated body (10), wherein the module control unit (21) serves to communicate the working parameters of the wand portion (20) to the main control unit (67), wherein the working parameters are selected from output voltage, current to the tip, timing of the function, and acoustical and optical indications for giving user feedback.

According to a second aspect, the present invention provides a kit-of-parts for providing a modular cord-less pen-style dental instrument of the present invention, comprising:

> (A) at least two wand portions as defined by claim 1, having a proximal end adapted to be releasably connected to the connector portion at the distal end of the elongated body, whereby at least two wand portions are provided having a functionality which is different from each other.

The present invention is based on the recognition that a cord-less pen-style dental instrument may be provided with a wide range of functionalities when a modular is selected provided that a heat flow barrier is present between the elongated body and the wand portion in order to reduce the accumulation of heat in the elongated body which may deteriorate the coupling of and the communication with a further wand portion when a further wand portion is used, in particular when the rotation around the longitudinal axis of the wand portion of the dental instrument must be maintained.

Furthermore, the present invention is based on the recognition that it is necessary to provide computational capabilities not only based on a microprocessor in the elongated body, but also in the wand portion so that a wide range of functionalities may be provided while at the same time a suitable standardized interface with a reduced number of electrical lines between the elongated body portion and the wand portion may be realized.

Brief Description of the Drawings

> Fig. 1A shows a cross-sectional top view of an embodiment of the modular cord-less pen-style dental instrument according to the present invention.
> Fig. 1B shows a detail of the modular cord-less pen-style dental instrument shown in Fig. 1A.
> Fig. 2 shows a top cross-sectional side view of the embodiment of the modular cord-less pen-style dental instrument according to the present invention as shown in Fig. 1.
> Fig. 3 shows a bottom view of an embodiment of the modular cord-less pen-style dental instrument according to the present invention as shown in Fig. 1.
> Fig. 4 shows a top view of an embodiment of the modular cord-less pen-style dental instrument according to the present invention as shown in Fig. 1.
> Fig. 5 shows an exploded drawing comprising the elongated body of the embodiment shown in Fig. 1.
> Fig. 6 shows a side view, Fig.7 a top-side view of the elongated body for gripping and holding the instrument.
> Fig. 8 shows a detailed side view, Fig. 9 shows a bottom-side view and Fig. 10 shows a less-detailed side view of the wand portion having a proximal end adapted to be releasably connected to the connector portion at the distal end of the elongated body.
> Fig. 11 shows a exploded top-side view of a battery module located inside the elongated body at the proximal end thereof.

Fig. 12 shows a side view and Fig. 13 a top-side view of an assembled battery module located inside the elongated body at the proximal end thereof.

Fig. 14 shows a cross-section, Fig. 15 a top view and Fig. 16 a top-side view of the charger base of the modular cord-less pen-style dental instrument.

**Detailed Description of Preferred Embodiments**

The present invention provides a modular cord-less pen-style dental instrument. The modular dental instrument provides multiple functionalities by a plurality of exchangeable wand portions. Each exchangeable wand portion is dedicated to a module specific function. The multiple functions of the dental instrument as defined in the present disclosure are achieved independently or in combination with a further wand portion of an additional modular cord-less pen-style dental instrument. The wand portion is releasably and communicatively connected via the connector portion to the elongated body. The elongated body provides power to the wand portion and controls the wand portion via the main control unit. For this purpose, the wand portion is provided with a module control unit. Furthermore, additional accessories could be attached to the said wand portions to achieve further functions.

The term "modular" means that the dental instrument is constructed with standardized units or dimensions for flexibility and variety in use. Accordingly, the proximal end of each wand portion is standardized, so that different wand portions of dedicated module specific functions can be assembled to a standardized elongated body via a standard connector portion. The assembled wand portion and the elongated portion as a whole has sufficient mechanical property and is fully functional for operation. Namely, the elongated body provides power to the wand portion and control the wand portion to operate via the control units. The multiple functions of the dental instrument as defined in the present disclosure are achieved independently or collaboratively by the plurality of exchangeable wand portions assembled to the elongated body.

The term "cord-less" means that the dental instrument does not require a wired external power supply during use. Accordingly, the dental instrument is typically powered by a battery such as a rechargeable battery, a stack of AA batteries, or supercapacitors. The term "cord-less" does not exclude the presence of an interface such as a plug where an electrical cable may be attached, for example for updating software or for repairing the dental instrument. The term "cord-less" does not exclude the presence of an interface such as a plug where an electrical cable may be attached to allow the use of an external power supply, for example in case the battery is out of order. In a preferred embodiment, the dental instrument comprises a wireless communication unit that can transmit and receive data via wireless transmission techniques, for example Bluetooth® or infra-red transmission technique. In another preferred embodiment, the dental instrument comprises a wireless power unit, which receive the electrical power via the near-field or far-field wireless power transmission for charging or operation.

The term "pen-style" means with regard to the dental instrument of the present invention that the overall shape and dimensions as well as the direction in which the functionality is delivered by the dental instrument, are adapted to that the dental practitioner may grasp, hold and operate the dental instrument with a single hand like a pen. Accordingly, the dental instrument of the present invention may have an overall length in the range of from 10 to 30 cm, more preferably 15 to 25 cm. The term "pen-style" does not specifically require the practitioner to grasp, hold, and operate the dental instrument exactly like a pen. The assembled modular pen-style dental instrument delivers the module specific function at the distal end of the wand portion and allows the practitioner to grasp, hold, and operate the dental instrument at around the centre or the proximal end of the assembled dental instrument. In a preferred embodiment, a switch is located on the side of the elongated body near the centre of the assembled dental instrument to allow the practitioner to control the function of the wand portion with a single hand.

The modular cord-less pen-style dental instrument of the present invention provides an elongated body for gripping and holding the instrument. The elongated body has in general a cylinder shape, which is suitable for the practitioner to grasp, hold and operate the dental instrument with a single hand. The elongated body provides space to accommodate essential components inside the body. In a preferred embodiment, a switch is located on the side of the elongated body near the centre of the assembled dental instrument to allow the practitioner to control the function of the wand portion with a single hand.

The elongated body has a connector portion at the distal end thereof. The connector portion provide communicative and electrical connection to the main control unit and the rechargeable battery inside the elongated body. The connector portion is standardized to allow the secure communicative, electrical, and mechanical connection to the proximal end of different wand portions. Consequently, the connector portion enable the secure, flexible, releasable, mechanical and electrical connection between the wand portion and the elongated body, so that the assembled dental instrument and be fully functional. Furthermore, the connector portion provide thermal insulation via a heat flow barrier between the wand portion and the elongated body. The thermal insulation prevents the heat generated in the wand portion to negatively affect the secure connection between the wand portion and the elongated body. The thermal insulation also prevents the heat to negatively affect the essential components in the elongated body. In a preferred embodiment, the connector portion allows the 360° rotation of the wand portion around the longitudinal axis of the dental instrument. The ability of rotation eases the practitioner in the operation of the dental instrument by providing an extra degree of freedom.

The modular cord-less pen-style dental instrument of the present invention provides a wand portion having a proximal end adapted to be releasably connected to the connector portion at the distal end of the elongated body. The wand portion is in general a cylinder-shaped object which delivers the module specific functionality at its distal end. The wand portion requires to be connected to the elongated body to be fully functional. The wand portion comprises module specific essential components inside its body. These essential components of the wand portion are to be connected to the control units in the elongated body to be controlled electrically powered. The wand portion has a standardized proximal end, which can be mechanically, communicatively, and electrically connected to the elongated portion via the connector portion. The wand portion comprises other essential components inside its body, which are not necessarily to be connected to the elongated body.

The dental instrument further comprises a heat flow barrier between the elongated body and the wand portion in the connector portion, which is located at the distal end of elongated body. A heat flow barrier prevents, reduces, or limits the heat transfer across the heat flow barrier passively or actively, when two sides of the heat flow barrier has a temperature difference.

Heat flow barrier may be provided by adjusting the possible temperature difference between the wand portion and the elongated body portion, the material present between the wand portion and the elongated body portion, the area through which the heat will be transferred between the wand portion and the elongated body portion, and the distance it must be transferred between the wand portion and the elongated body portion.

Accordingly, assuming a flat surface interface, the transfer of heat from a wand portion with a temperature of T1 to an elongated body portion with a temperature of T2 through a surface area A of the heat flow barrier having a thickness d, whereby the thermal conductivity value of the heat flow barrier is $k$, the equation relating the heat transfer rate to these variables is

$$\text{Rate} = k \cdot A \cdot (T1 - T2)/d \qquad \text{(Joule/second)}$$

Preferably, the material of the heat flow barrier is a thermal insulator having a thermal conductivity k of the material of at most 1.5 W/m/°C, preferably at most 1 W/m/°C, more preferably at most 0.1 W/m/°C.

The heat flow barrier prevents the heat flow from negatively affecting the secure connection between the wand portion and the elongated body. The heat flow barrier also prevents the heat flow to negatively affect the essential components in the elongated body or the wand portion. In a preferred embodiment, the heat flow barrier thermally isolates the wand portion from the elongated body to prevent overheating of the electronics and connector portion by heat flow generated by the wand portion. In another preferred embodiment, the heat flow barrier provides thermal insulation between the wand portion and the elongated body to prevent undesired heating of the cooled wand portion by the heat flow generated from the battery, warm hand, or the control units. In yet another preferred embodiment, the heat flow barrier provides active cooling to avoid the overheating of the connection portion, which would otherwise lead to insecure connection of the wand portion and the elongated body.

The modular pen-style dental instrument as defined in the present disclosure offers the wand portion provides functionality selected from

    (i) radiating curing light radiation;
    (ii) radiating diagnostic illumination;
    (iii) providing illumination for cancer screening;
    (iv) providing self-cleaning indirect vision by rotating a dental mirror;
    (v) vibrating a dental tip
    (vi) providing pulp vitality testing based on current stimulation or heat/cold application;
    (vii) providing heated Gutta percha plugging; and
    (viii) facilitating fluorescence aided caries detection.

The functionality of radiating curing light radiation refers to the electromagnetic radiation that is suitable for curing a dental composition. The radiation needs to have enough intensity and has a peak wavelength that is compatible with the specific light curable dental composition. Typically, the wavelength of the curing light may be in the range of violet and/or blue light. The light source of the radiation is selected from light emitting diodes (LED) having an emission maximum in the desired range. In a preferred embodiment, a range of wand portions that emit curing light at different wavelengths and/or different intensity are provided so that the practitioner can choose a most suitable wand portion for a specific light curable dental composition.

The functionality of radiating diagnostic illumination refers to the electromagnetic radiation that is suitable for illuminating the oral cavity. Preferably a bright white light is used. The light source at the distal end of the wand portion may illuminate the oral cavity, so that treatment and/or diagnosis can be performed based on the light from reflectance, fluorescence, and/or transmission.

The functionality of providing illumination for cancer screening refers to a case where the light source at the distal end of the wand portion can emit light at a specific wavelength suitable for visually distinguishing a cancerous tissue from normal tissues by a difference in the fluoresce and/or reflection, so that the dental instrument can be used for oral cancer screening.

The functionality of self-cleaning indirect vision by rotating a dental mirror refers to a mirror refers to a self-cleaning dental mirror, which is incorporated at the distal end of the wand portion to provide the practitioner with indirect vision. The self-cleaning function is achieved by including a rotating dental mirror, which utilizes centrifugal force to remove the water droplets from water spray or saliva which could otherwise block or blur the vison.

The functionality of vibrating a dental tip refer to a tip at the distal end of the wand portion which is adapted to provide vibrational, e.g. ultrasonic vibrational, capabilities. The dental tip may be selected from a polishing brush, an ultrasonic tip, or a needle. Certain highly viscous dental compositions are provided with thixotropic properties, so that the application of ultrasound vibration facilitates the flow of the dental composition into a dental cavity. In a preferred embodiment, an ultrasonic tip at the distal end of the wand portion can be used to deliver ultrasound vibration to improve the flowability of a highly viscous dental composition. In one embodiment, the vibrating tip is used for cleaning, in particular rinsing, of a root canal. The vibration of the tip or needle, which is inserted into the root canal, leads to turbulent fluctuations of the rinsing solution, which has been introduced into the root canal before, resulting in improved cleaning of the canal.

The functionality of providing pulp vitality testing based on current stimulation or heat/cold application, refers to a tip at the distal end of the wand portion which is adapted to provide heating/cooling or current conducting capabilities. Pulp vitality can be tested via an electrical current, cooling or heating. When a current, cold source, or hot source is applied to the tooth, a neurological response is elicited and is reported by the patient. A wand portion with heating/cooling or current conducting ability are suitable for such pulp vitality testing.

The functionality of providing heated Gutta percha plugging refers to a tip at the distal end of the wand portion which is adapted to provide heating capabilities. Gutta percha is a rubber-like elastomer made of monomer unit as trans-isoprene. It is typically used during root canal therapy. Warmed or heated Gutta percha plug is used for consistently dense, dimensionally stable, three-dimensional root canal fillings. A wand portion with a heating tip allowed the process of heated Gutta percha plugging.

The functionality of facilitating fluorescence aided caries detection refers to the irradiation of light at a wavelength and intensity which is able to visually distinguish carious lesions from healthy hard dental tissue.

The heat flow barrier in the modular pen-style dental instrument as defined in the present disclosure may comprise is an air-gap and/or one or more solid elements comprising a heat-insulation material. The heat flow barrier can be provided by using a combination of thermal insulator as defined above, selected from air-gap, a vacuum gap, and/or one or more solid elements comprising a heat-insulation material. The solid elements comprising the heat-insulation material may be in the form of solid bodies, closed-cell foams, open-cell foams, non-woven fabrics, porous materials, or a combination thereof.

The heat flow barrier in the modular pen-style dental instrument as defined in the present disclosure wherein the one or more solid elements comprise a rubber O-ring and parts of the elongated body and the wand portion which are in contact with each other, which are made of a resin. Preferably, the heat flow barrier is an O-ring or a cylinder that has a hole along its axis, which allow the electrical connection to pass through the heat flow barrier. In a preferred embodiment, the heat flow barrier comprises an air gap and an O-ring made from a rubber or other suitable resin. The heat flow barrier may also comprise a thermo composite containing phase changing materials.

The heat flow barrier may also function collaboratively with the components in the wand portion or elongated body, or external accessories that is mounted to the dental instrument. In a preferred embodiment, the heat flow barrier is a thermoelectric unit powered by the control unit, that actively cool the connector portion. In another preferred embodiment, the heat flow barrier comprises a heat sink, which is continuously cooled by a coolant that is circulated by pumping or convection.

In the present disclosure, the elongated body comprises a rechargeable battery and a main control unit communicatively connected through the connector portion with the wand portion, or by using a wireless communication. The elongated body comprises a main control unit, a rechargeable battery, and a connector portion. The main control unit is an electrical system that comprise circuits and one or more chips. The primary electronics for the main control unit are generally mounted on a conventional printed circuit board (either rigid or flexible). A communicatively connection refers to an electrical connection that transmit both power and signals, so that the connected parts can be powered and/or communicate with each other. Preferably, the main control unit is communicatively connected with the wand portion via a wireless connection, such as blue-tooth connection. The rechargeable battery is communicatively and electrically connected to the main control unit. The rechargeable battery may be selected from a rechargeable battery pack, one or more AA batteries, or a supercapacitor pack.

Preferably, a switch is installed on the outside of the elongated body, and the switch is communicatively connected to the main control unit. The switch enables the activation of a specific function of the wand portion.

Preferably in the present disclosure, the main control unit is communicatively connected through the connector portion with the wand portion *via* a single data line. The single data line refers to a single conductive wire that can transmit signal. Preferably, a further power line and optionally a ground line are present. The merit of a single data line connection is to allow a simple and robust electrical connection between two parts that can freely rotate. Preferably, the connection through the connector portion with the wand portion shows rotational symmetry around the longitudinal axis of the wand portion.

In the present disclosure, the wand portion comprises a module control unit powered by the rechargeable battery in the elongated body and communicatively connected with the main control unit of the elongated body.

In the present disclosure, the wand portion comprises a module control unit, wherein the module control unit serves to communicate the working parameters of the wand portion to the main control unit, wherein the working parameters are selected from output voltage, current to the tip, timing of the function, and acoustical and optical indications for giving user feedback.

In the present disclosure, the proximal end of the wand portion is releasably connected to the connector portion at the distal end of the elongated body. Preferably, the connection is achieved by a friction fit connection or form fit connection, or a combination thereof. A friction fit is a type of interference fit, which refers to a fastening between two parts which is achieved by friction after the parts are pushed together, rather than by any other means of fastening. A form fit refers to a connection a fastening between two parts which is achieved by the shape change.

More preferably, the wand portion is releasably connected to the elongated body by a quick-coupler connection. A quick-coupler connection refers to a connection that allows the rapid change of attachments. They also bring with them additional safety risks that must be overcome by careful design and manufacture, and proper use. Even more preferably, the quick coupler connection allows 360° rotation of the wand around the longitudinal axis of the instrument.

In the present disclosure, the modular cord-less pen-style dental instrument is provided in the form of kit-of-parts. These modules may be implemented in a variety of embodiments and can be packaged in a number of configurations without departing from the scope of the invention as set forth in the claims.

The kit-of-parts comprises (A) at least two wand portions, having a proximal end adapted to be releasably connected to the connector portion at the distal end of at least one of the elongated bodies, whereby at least two wand portions provided a functionality which different from each other.

Preferably, the kit-of-parts further comprises one or more elongated body for gripping and holding the instrument, which have a connector portion at the distal end thereof.

Preferably, the kit-of-parts for providing a modular cord-less pen-style dental instrument, which further comprises (C) a charger base. The charger base has one or more housings which is provided with means for supporting and receiving components or the assembled components of the dental instruments as defined in the present disclosure. The charger base provides a light intensity testing region to test.

The present invention will now be further described based on the figures.

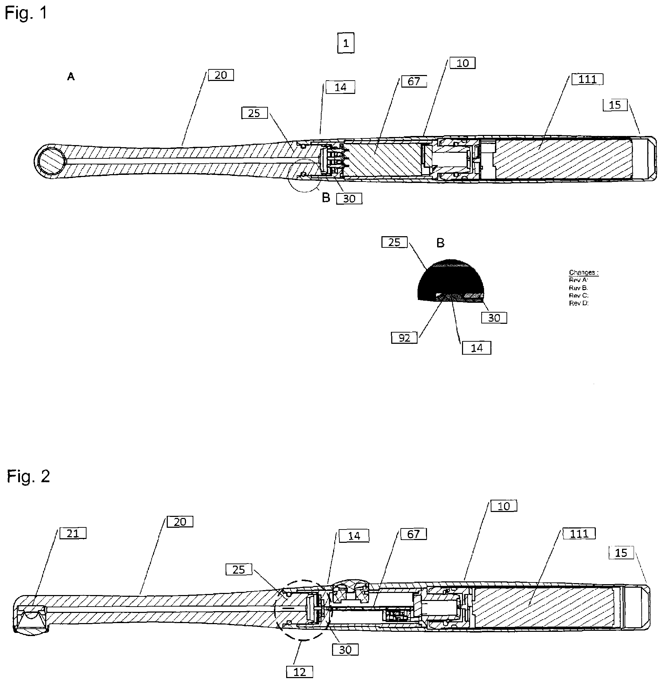

Fig. 1A shows a cross-sectional top view of an embodiment of the modular cord-less pen-style dental instrument 1 according to the present invention. Specifically, Fig. 1A shows the elongated body 10 for gripping and holding the instrument and the distal end 14 and the proximal end 15 of the elongated body. The elongated body 10 contains a main control unit 67 and a rechargeable battery 111. Fig. 1A shows a wand portion 20 and the proximal end thereof 25. The connection between elongated body 10 and the wand portion 20 is thermally insulated by a heat flow barrier 30.

Fig. 1B depicts the connector parts of the elongated body 10 and the wand portion 20 from Fig. 1A in greater detail. The distal end 14 of the elongated body 10, the proximal end of the wand portion 25, the heat flow barrier 30 and a rubber O-ring 92, which supports 360° rotation around the longitudinal axis of the dental instrument 1, and heat insulation between the elongated body 10 and the wand portion 20, is shown.

Fig. 2 shows a top cross-sectional side view of the embodiment of the modular cord-less pen-style dental instrument according to the present invention as shown in Fig. 1. Specifically, Fig. 2 shows the elongated body 10 for gripping and holding the instrument and the distal end 14 and the proximal end 15 of the elongated body. The elongated body 10 contains, a main control unit 67 and a rechargeable battery 111. Fig. 2 shows a wand portion 20 and the module control unit 21 thereof as well as the proximal end 25. The connection between elongated body 10 and the wand portion 20 is established by a connector portion 12 at the distal end of the elongated body 12 that is thermally insulated by a heat flow barrier 30.

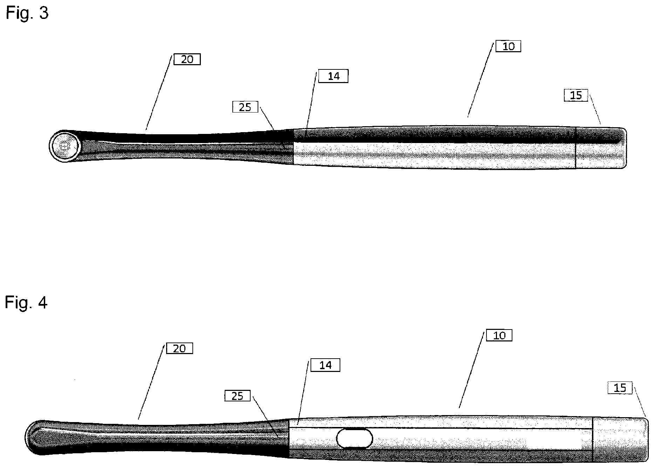

Fig. 3 shows a bottom view of an embodiment of the modular cord-less pen-style dental instrument according to the present invention as shown in Fig. 1. Specifically, an elongated body 10 and the distal end 14 and the proximal end 15 thereof is shown. The proximal end 25 of the wand portion 20 is seamlessly connected to the elongated body 10 and supports 360° rotation around the longitudinal axis of the dental instrument 1.

Fig. 4 shows a top view of an embodiment of the modular cord-less pen-style dental instrument according to the present invention as shown in Fig. 1. Specifically, an elongated body 10 and the distal end 14 and the proximal end 15 thereof is shown. The proximal end 25 of the wand portion 20 is seamlessly connected to the elongated body 10 and supports 360° rotation around the longitudinal axis of the dental instrument 1.

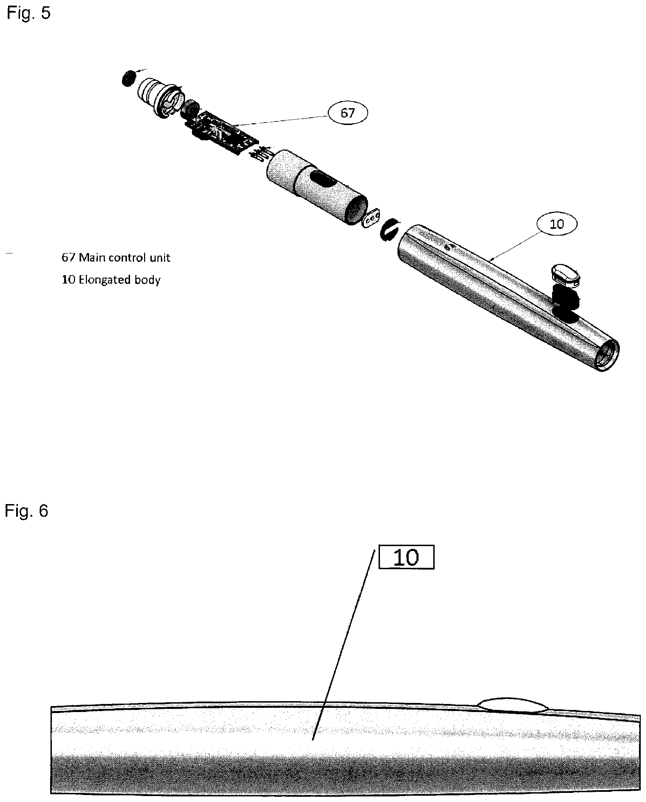

Fig. 5 shows the individual interior parts comprising elongated body 10 of the embodiment shown in Fig. 1.

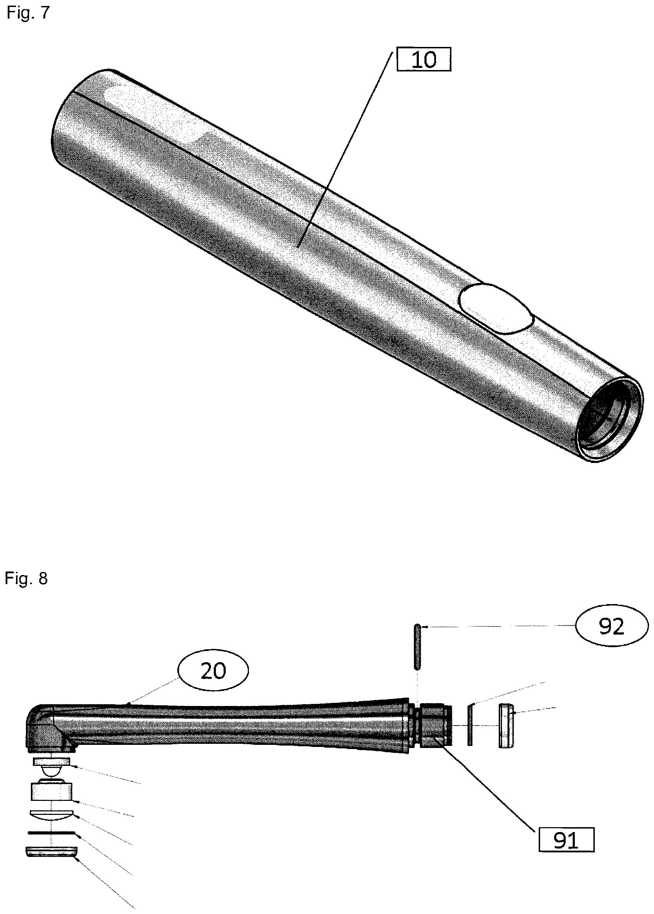

Fig. 6 shows a side view, Fig.7 a top-side view of the elongated body 10 for gripping and holding the instrument.

Fig. 8 shows a detailed side view of the wand portion 20 having a proximal end 25 adapted to be releasably connected to the connector portion 12 at the distal end 14 of the elongated body 10. Specifically, the wand portion 20 and the quick-coupler connection 91 thereof is shown. The quick-coupler connection 91 supports 360° rotation around the longitudinal axis of the dental instrument 1, and the contact between the wand portion 20 and the elongated body 10 is established by a rubber O-ring 92.

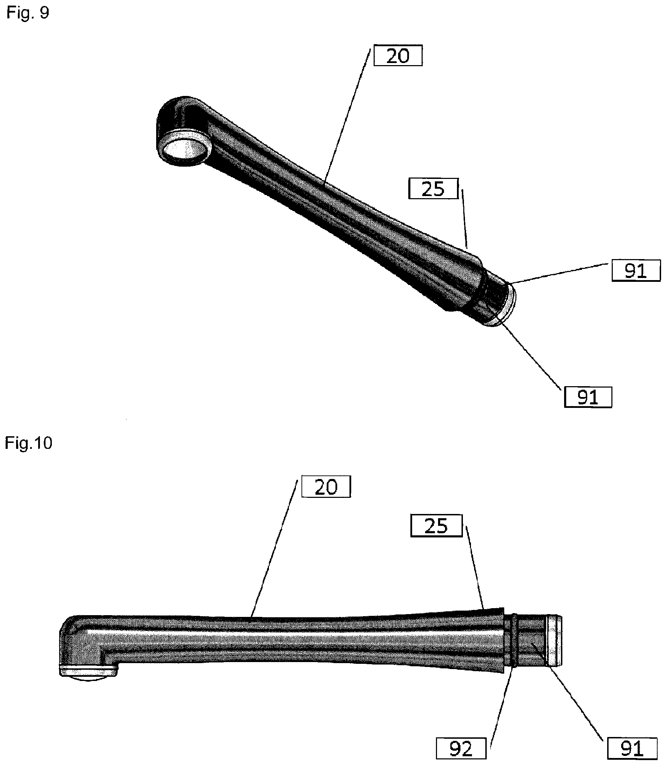

Fig. 9 shows a bottom-side view and Fig. 10 shows a less-detailed side view of the embodiment described in Figure 8.

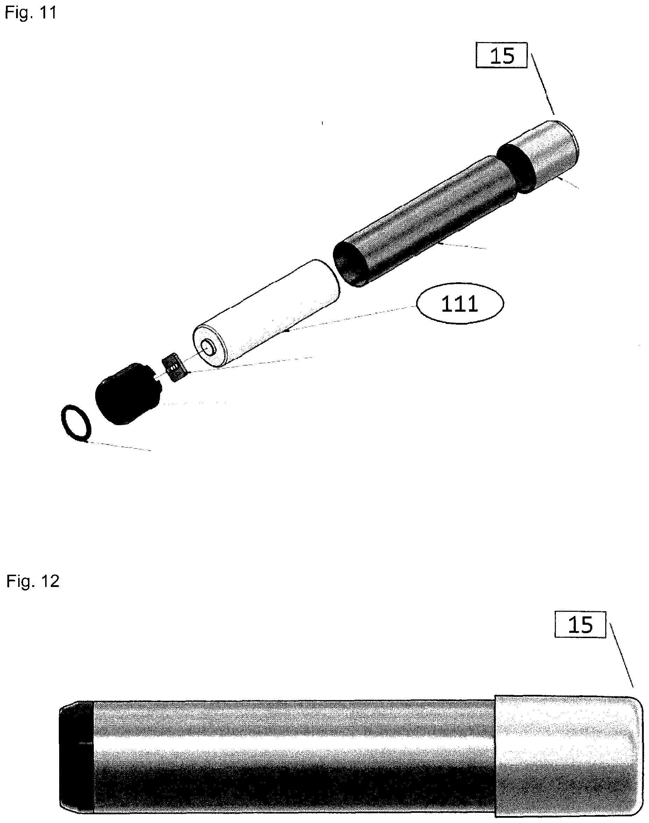

Fig. 11 shows an exploded top-side view of a disassembled battery module located inside the elongated body at the proximal end thereof. Specifically, the proximal end 15 of the elongated body 10 is shown. A rechargeable battery 111 is shown.

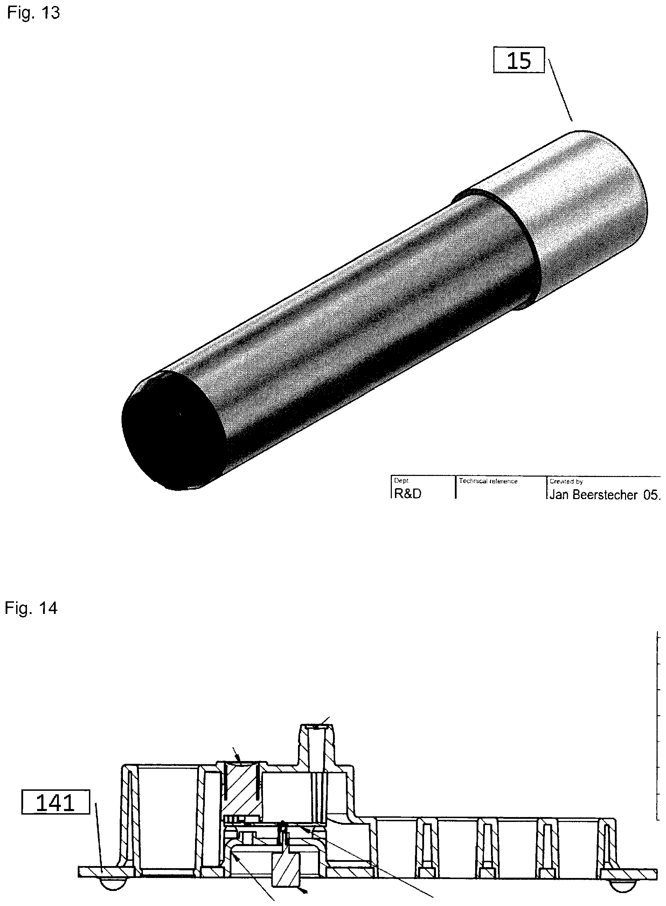

Fig. 12 shows a side view and Fig. 13 a top-side view of an assembled battery module located inside the elongated body at the proximal end thereof. Specifically, the proximal end of the elongated body 15 is shown, which establishes the rear end of the modular cord-less pen-style dental instrument 1.

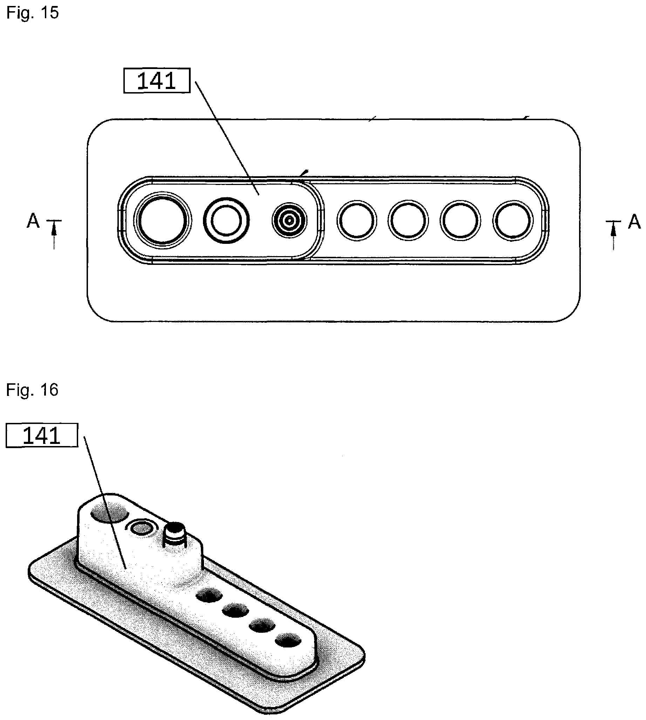

Fig. 14 shows a cross-section, Fig. 15 a top view and Fig. 16 a top-side view of the charger base of the modular cord-less pen-style dental instrument. Specifically, a charger base 141 is shown.

List of Reference Numerals

| | |
|---|---|
| 1: | modular cord-less pen-style dental instrument comprising: |
| 10: | an elongated body |
| 12: | connector portion at the distal end of the elongated body |
| 14: | distal end of elongated body |
| 15: | proximal end of elongated body |
| 20: | a wand portion |
| 21: | module control unit |
| 25: | proximal end of wand portion |
| 30: | heat flow barrier |
| 67: | main control unit |
| 91: | quick-coupler connection |
| 92: | rubber O-ring |
| 111: | rechargeable battery |
| 141: | charger base |

**Claims**

1. A modular cord-less pen-style dental instrument (1) comprising:

   (a) an elongated body (10) for gripping and holding the instrument, which has a connector portion (12) at the distal end thereof; and
   (b) a wand portion (20) having a proximal end adapted to be releasably connected to the connector portion (12) at the distal end of the elongated body,
   wherein the connector portion provides thermal insulation via a heat flow barrier (30) between the wand portion (20) and the elongated body (10) to reduce the accumulation of heat in the elongated body (10);
   wherein the elongated body (10) comprises a rechargeable battery (111) and a main control unit (67) communicatively connected through the connector portion (12) with the wand portion (20), and
   the wand portion (20) comprises a module control unit (21) powered by the rechargeable battery (111) in the elongated body (10) and communicatively connected with the main control unit (67) of the elongated body (10),
   **characterized in that**,
   the module control unit (21) serves to communicate the working parameters of the wand portion (20) to the main control unit (67), wherein the working parameters are selected from output voltage, current to the tip, timing of the function, and acoustical and optical indications for giving user feedback.

2. The modular pen-style dental instrument according to claim 1, wherein the wand portion provides functionality selected from

(i) radiating curing light radiation;
(ii) radiating diagnostic illumination;
(iii) providing illumination for cancer screening;
(iv) providing self-cleaning indirect vision by rotating a dental mirror;
(v) vibrating a dental tip
(vi) providing pulp vitality testing based on current stimulation or heat/cold application;
(vii) providing heated Gutta percha plugging; and
(viii) facilitating fluorescence aided caries detection.

3. The modular pen-style dental instrument (1) according to claim 1 or 2,
wherein the heat flow barrier (30) is an air-gap and/or one or more solid elements comprising a heat insulation material.

4. The modular pen-style dental instrument according to claim 1, wherein the one or more solid elements comprise a rubber O-ring (92) and parts of the elongated body (10) and the wand portion (20) which are in contact with each other, which are made of a resin.

5. The modular pen-style dental instrument according to claim 1, wherein the main control unit (67) is communicatively connected through the connector portion (12) with the wand portion (20) *via* a single line.

6. The modular pen-style dental instrument according to any one of the preceding claims, wherein the proximal end of the wand portion (25) is releasably connected to the connector portion (12) at the distal end of the elongated body by a friction fit connection or form fit connection, or a combination thereof.

7. The modular pen-style dental instrument according to any one of the preceding claims, wherein the wand portion (20) is releasably connected to the elongated body (10) by a quick-coupler connection (91).

8. The modular pen-style dental instrument according to claim 7, wherein the quick coupler connection (91) allows 360° rotation of the wand around the longitudinal axis of the instrument.

9. Kit-of-parts for providing a modular cord-less pen-style dental instrument as defined by any one of the preceding claims, comprising:

(A) at least two wand portions (20) as defined by claim 1, having a proximal (25) end adapted to be releasably connected to the connector portion (12) at the distal end (14) of the elongated body, whereby at least two wand portions are provided having a functionality which is different from each other.

10. The kit-of-parts according to claim 9, which further comprises
(B) one or more elongated bodies (10) for gripping and holding the instrument, which have a connector portion (12) at the distal end thereof;

11. The kit-of-parts for providing a modular cord-less pen-style dental instrument according to claim 9 or 10, which further comprises
(C) a charger base (141).

**Patentansprüche**

1. Modulares kabelloses zahnärztliches Instrument in Stiftform (1), bestehend aus:

(a) einen länglichen Körper (10) zum Greifen und Halten des Instruments, der an seinem distalen Ende einen Verbindungsabschnitt (12) aufweist; und
(b) ein Stabteil (20) mit einem proximalen Ende, das so angepasst ist, dass es lösbar mit dem Verbindungsteil (12) am distalen Ende des länglichen Körpers verbunden werden kann,

wobei der Verbindungsabschnitt eine thermische Isolierung über eine Wärmestrombarriere (30) zwischen dem Stababschnitt (20) und dem länglichen Körper (10) bereitstellt, um die Ansammlung von Wärme in dem länglichen Körper (10) zu reduzieren;

wobei der langgestreckte Körper (10) eine wiederaufladbare Batterie (111) und eine Hauptsteuereinheit (67) umfasst, die über den Verbindungsabschnitt (12) mit dem Stababschnitt (20) kommunikativ verbunden ist, und

der Stababschnitt (20) eine Modulsteuereinheit (21) umfasst, die von der wiederaufladbaren Batterie (111) in dem langgestreckten Körper (10) mit Strom versorgt wird und mit der Hauptsteuereinheit (67) des langgestreckten Körpers (10) kommunikativ verbunden ist, **dadurch gekennzeichnet, dass** die Modulsteuereinheit (21) dazu dient, die Arbeitsparameter des Zauberstabschnitts (20) an die Hauptsteuereinheit (67) zu übermitteln, wobei die Arbeitsparameter aus der Ausgangsspannung, dem Strom zur Spitze, dem Zeitablauf der Funktion und akustischen und optischen Anzeigen zur Rückmeldung an den Benutzer ausgewählt werden.

2. Modulares zahnärztliches Instrument in Stiftform nach Anspruch 1, wobei der Stabteil eine Funktionalität bietet, die ausgewählt ist aus

   (i) strahlt härtende Lichtstrahlung aus;
   (ii) strahlt diagnostisches Licht aus;
   (iii) mit Beleuchtung für die Krebsvorsorge;
   (iv) Ermöglichung einer selbstreinigenden indirekten Sicht durch Drehen eines Zahnarztspiegels;
   (v) Vibration einer Zahnspitze
   (vi) bietet Vitalitätstests für das Fruchtfleisch auf der Grundlage von Stromstimulation oder Wärme-/Kälteanwendungen
   (vii) mit beheiztem Guttapercha-Stopfen; und
   (viii) zur Erleichterung der fluoreszenzgestützten Karieserkennung.

3. Modulares zahnärztliches Stiftinstrument (1) nach Anspruch 1 oder 2, wobei die Wärmestrombarriere (30) ein Luftspalt und/oder ein oder mehrere feste Elemente ist, die ein wärmeisolierendes Material umfassen.

4. Modulares zahnärztliches Stiftinstrument nach Anspruch 1, wobei das eine oder die mehreren festen Elemente einen Gummi-O-Ring (92) und Teile des länglichen Körpers (10) und des Schaftteils (20), die miteinander in Kontakt sind, umfassen, die aus einem Harz hergestellt sind.

5. Modulares stiftförmiges zahnärztliches Instrument nach Anspruch 1, wobei die Hauptsteuereinheit (67) über den Verbindungsabschnitt (12) mit dem Schaftabschnitt (20über eine einzige Leitung kommunikativ verbunden ist.

6. Modulares zahnärztliches Instrument in Stiftform nach einem der vorhergehenden Ansprüche, wobei das proximale Ende des Stabteils (25) mit dem Verbindungsteil (12) am distalen Ende des länglichen Körpers durch eine Reibschlussverbindung oder eine Formschlussverbindung oder eine Kombination davon lösbar verbunden ist.

7. Modulares zahnärztliches Instrument in Stiftform nach einem der vorhergehenden Ansprüche, wobei der Schaftteil (20) durch eine Schnellkupplungsverbindung (91) lösbar mit dem länglichen Körper (10) verbunden ist.

8. Modulares zahnärztliches Instrument in Stiftform nach Anspruch 7, wobei die Schnellkupplungsverbindung (91) eine 360°-Drehung des Stabes um die Längsachse des Instruments ermöglicht.

9. Teilesatz zur Bereitstellung eines modularen kabellosen stiftförmigen Dentalinstruments nach einem der vorhergehenden Ansprüche, umfassend:

   (A) mindestens zwei Stababschnitte (20) gemäß Anspruch 1, die ein proximales (25) Ende aufweisen, das geeignet ist, lösbar mit dem Verbindungsabschnitt (12) am distalen Ende (14) des länglichen Körpers verbunden zu werden, wobei mindestens zwei Stababschnitte mit einer voneinander verschiedenen Funktionalität vorgesehen sind.

10. Der Teilesatz nach Anspruch 9, der außerdem Folgendes umfasst
    (B) ein oder mehrere längliche Körper (10) zum Greifen und Halten des Instruments, die am distalen Ende einen Verbindungsabschnitt (12) aufweisen;

**11.** Teilesatz zur Herstellung eines modularen kabellosen stiftförmigen zahnärztlichen Instruments nach Anspruch 9 oder 10, der ferner umfasst
(C) eine Ladestation (141).

**Revendications**

**1.** Instrument dentaire modulaire sans fil de type stylo (1) comprenant :

(a) un corps allongé (10) pour la saisie et le maintien de l'instrument, qui présente une partie connecteur (12) à l'extrémité distale de celui-ci ; et
(b) une partie tige (20) présentant une extrémité proximale adaptée pour être reliée de manière amovible à la partie connecteur (12) à l'extrémité distale du corps allongé,
dans lequel la partie connecteur fournit une isolation thermique via une barrière de flux thermique (30) entre la partie tige (20) et le corps allongé (10) pour réduire l'accumulation de chaleur dans le corps allongé (10) ;
dans lequel le corps allongé (10) comprend une batterie rechargeable (111) et une unité de commande principale (67) reliée en communication à travers la partie connecteur (12) à la partie tige (20), et
la partie tige (20) comprend une unité de commande de module (21) alimentée par la batterie rechargeable (111) dans le corps allongé (10) et reliée en communication à l'unité de commande principale (67) du corps allongé (10), **caractérisé en ce que** l'unité de commande de module (21) sert à communiquer les paramètres de fonctionnement de la partie tige (20) à l'unité de commande principale (67), dans lequel les paramètres de fonctionnement sont sélectionnés parmi la tension de sortie, le courant à l'extrémité, le moment d'exécution de la fonction et les indications acoustiques et optiques pour donner un retour à l'utilisateur.

**2.** Instrument dentaire modulaire sans fil de type stylo selon la revendication 1, dans lequel la partie tige fournit une fonctionnalité sélectionnée parmi

(i) l'émission d'un rayonnement lumineux de durcissement ;
(ii) l'émission d'un éclairage de diagnostic ;
(iii) la fourniture d'un éclairage pour le dépistage du cancer ;
(iv) la fourniture de vision indirecte autonettoyante par rotation d'un miroir dentaire ;
(v) la vibration d'une pointe dentaire
(vi) la fourniture d'un test de vitalité des pulpes sur la base de la stimulation par courant ou de l'application de chaleur/froid ;
(vii) la fourniture d'un colmatage avec de la gutta-percha chauffée ; et
(viii) la facilitation de la détection de caries par fluorescence.

**3.** Instrument dentaire modulaire sans fil de type stylo (1) selon la revendication 1 ou 2, dans lequel la barrière de flux de chaleur (30) est une fente à air et/ou un ou plusieurs éléments solides comprenant un matériau thermoisolant.

**4.** Instrument dentaire modulaire sans fil de type stylo selon la revendication 1, dans lequel les un ou plusieurs éléments solides comprennent un joint torique (92) en caoutchouc et des parties du corps allongé (10) et la partie tige (20) qui sont en contact les unes avec les autres, qui sont réalisées en une résine.

**5.** Instrument dentaire modulaire sans fil de type stylo selon la revendication 1, dans lequel l'unité de commande principale (67) est reliée en communication à travers la partie connecteur (12) à la partie tige (20) via une seule ligne.

**6.** Instrument dentaire modulaire sans fil de type stylo selon l'une des revendications précédentes, dans lequel l'extrémité proximale de la partie tige (25) est reliée de manière amovible à la partie connecteur (12) à l'extrémité distale du corps allongé par un raccord à friction ou un raccord de formes, ou une combinaison de ceux-ci.

**7.** Instrument dentaire modulaire sans fil de type stylo selon l'une des revendications précédentes, dans lequel la partie tige (20) est reliée de manière amovible au corps allongé (10) par une liaison à couplage rapide (91).

**8.** Instrument dentaire modulaire sans fil de type stylo selon la revendication 7, dans lequel la liaison à couplage rapide (91) permet une rotation à 360° de la tige autour de l'axe longitudinal de l'instrument.

**9.** Kit de pièces pour fournir un instrument dentaire modulaire sans fil de type stylo selon l'une des revendications précédentes, comprenant :

(A) au moins deux parties tige (20) selon la revendication 1, présentant une extrémité proximale (25) adaptée pour être reliée de manière amovible à la partie connecteur (12) à l'extrémité distale (14) du corps allongé, selon lequel au moins deux parties tige sont prévues avec une fonctionnalité

qui est différente l'une de l'autre.

10. Kit de pièces selon la revendication 9, qui comprend en outre
(B) un ou plusieurs corps allongés (10) pour la saisie et le maintien de l'instrument, lesquels ont une partie connecteur (12) à l'extrémité distale de ceux-ci ;

11. Kit de pièces pour fournir un instrument dentaire modulaire sans fil de type stylo selon la revendication 9 ou 10, qui comprend en outre (C) une base de chargeur (141).

Fig. 1

Changes :
Rev A:
Rev B:
Rev C:
Rev D:

Fig. 2

Fig. 3

Fig. 4

Fig. 5

67 Main control unit
10 Elongated body

Fig. 6

Fig. 7

Fig. 8

Fig. 9

20

25

91

91

Fig.10

20

25

92

91

Fig. 11

Fig. 12

Fig. 13

15

| Dept | Technical reference | Created by |
|------|---------------------|------------|
| R&D | | Jan Beerstecher 05. |

Fig. 14

141

Fig. 15

141

A

A

Fig. 16

141

**EP 3 937 843 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006275732 A
- US 2005282102 A1